# EUROPEAN PATENT APPLICATION

(11) **EP 3 511 410 A1**
(43) Date of publication of application: **17.07.2019**
(21) Application number: 17848879.7
(22) Date of filing: 08.09.2017
(51) Int. Cl.: C12N 5/0735, C12N 5/071, C12N 5/0775, C12N 5/10

(54) **MEDIUM FOR CULTURING STEM CELLS, METHOD FOR CULTURING STEM CELLS, STEM CELL GROWTH PROMOTER, AND CELL COMPOSITION AND METHOD FOR PRODUCING SAME**

(30) Priority: 08.09.2016 JP 2016175941
(71) Applicant: Kyowa Hakko Bio Co., Ltd., Tokyo 100-8185 (JP); Kyowa Hakko Kirin Co., Ltd., Tokyo 100-8185 (JP)
(72) Inventor: YAMAGUCHI Kana, Tokyo 100-8185 (JP); SHOJI Shinichiro, Tokyo 100-8185 (JP); TSUKAHARA Masayoshi, Tokyo 100-8185 (JP)
(74) Representative: Vossius & Partner Patentanwälte Rechtsanwälte mbB
(86) International application number: PCT/JP2017/032456
(87) International publication number: WO 2018/047941

(57) **Abstract**

An object of the present invention is to provide a technique for stably, inexpensively, and safely culturing stem cells having a differentiation ability while maintaining an undifferentiated state. The present invention relates to a medium for culturing stem cells, comprising at least one or more compounds selected from the group consisting of a ROCK inhibitor, a PKC inhibitor, a histone methyltransferase inhibitor, and a retinoic acid receptor agonist, and not containing a growth factor or having a low growth factor concentration; a method for culturing stem cells using the medium; a growth promoter for stem cell; as well as a cell composition containing stem cells or differentiated cells therefrom; and a method for producing the cell composition.

## Description

### Technical Field

The present invention relates to a medium for culturing stem cells, a method for culturing stem cells using the medium, a growth promoter for stem cells, as well as a cell composition containing stem cells or differentiated cells therefrom and a method for producing the cell composition. More particularly, the invention relates to a medium for culturing stem cells characterized in that a compound capable of efficiently culturing stem cells is added to a medium which does not contain a growth factor or has a low growth factor concentration, and the like.

### Background Art

Culturing of stem cells is generally carried out using a medium supplemented with a growth factor such as serum replacement factor KSR (knockout-serum replacement) or fibroblast growth factor-2 (FGF-2), and mouse embryonic tissue-derived fibroblasts or the like as feeder cells. Alternatively, feeder cells are not used, and a culture container is coated with an extracellular matrix such as Matrigel, vitronectin, laminin, or fibronectin, or a recombinant protein or the like, and culture is carried out in a medium in which a growth factor such as a fibroblast growth factor (FGF) is added to a culture supernatant obtained by culturing feeder cells. In these culture operations, a component derived from a heterologous species may be incorporated, and also an unknown component may be incorporated at an unknown concentration.

If a component derived from a heterologous species is incorporated in this manner, it becomes a factor to make the culturing unstable, and moreover may cause infection with a virus or a pathogen common to humans and animals or may become a heterologous antigen. Thus, the component derived from a heterologous species is preferably removed from the culturing.

Therefore, at present, in order to culture stem cells as safely as possible, an extracellular matrix component such as vitronectin, laminin, or fibronectin or a recombinant thereof is used as a scaffolding material, and a serum-free medium whose components are all known (chemically defined serum-free medium) is used as a medium. As the medium, TeSR1 medium, TeSR-E8 medium, STEMPRO medium, hESF9 or hESF-FX medium, and the like are exemplified.

In a main composition of such a medium, high-glucose containing DMEM or DMEM/F12 (a medium obtained by mixing DMEM and F12 medium at 1:1) is used as a minimal essential medium, and an inorganic salt, an amino acid, a vitamin, a saccharide, or a minor component or the like is contained, and further, insulin, transferrin, albumin, a lipid group or the like, a growth factor or the like, or mercaptoethanol or the like is added.

As the growth factor to be added to the medium, for example, a basic fibroblast growth factor (bFGF) belonging to the FGF family, TGF-β (TGF-β₁) (PTL 1) and activin (PTL 2) belonging to the transforming growth factor-β family, heregulin-β-1 belonging to the neuregulin family and IFG-1 belonging to the insulin-like growth factor (IGF) family (NPL 1), and Wnt-3a belonging to the Wnt family are known.

### Citation List

### Patent Literature

PTL 1: WO 2004/055155
PTL 2: JP-A-2012-143229

### Non Patent Literature

NPL 1: Wang, L., et al. (2007), Blood 110, 4111-4119

### Summary of Invention

### Technical Problem

A growth factor as described above is expensive, and the biological activity varies depending on the manufacturer or the origin, and it may become a factor to make the medium unstable. Further, such a growth factor may be contaminated with a component derived from a heterologous species, a pathogen, or the like in its production process or the like. When it is used in medical application, it is necessary to confirm the biological origin. Therefore, it is preferred to substitute the growth factor by a component other than a protein, or to reduce the growth factor as much as possible, or to remove the growth factor.

Therefore, an object of the invention is to provide a technique for stably, inexpensively, and safely culturing stem cells having a differentiation ability while maintaining an undifferentiated state.

### Solution to Problem

The present inventors found that the above object can be achieved by using a medium obtained by adding a specific compound to a medium which does not contain a growth factor or has a low growth factor concentration, and thus completed the invention.

That is, the present invention relates to the following (1) to (20).
(1) A medium for culturing stem cells, comprising at least one or more compounds selected from the group consisting of a ROCK (Rho-associated coiled-coil forming kinase) inhibitor, a PKC (protein kinase C) inhibitor, a histone methyltransferase inhibitor, and a retinoic acid receptor agonist and not containing a growth factor or having a low growth factor concentration.
(2) The medium for culturing stem cells according to claim 1, wherein the growth factor is a basic fibroblast growth factor.
(3) The medium for culturing stem cells described in (1), wherein a concentration of the basic fibroblast growth factor is 2 ng/mL or less.
(4) The medium for culturing stem cells described in (2), wherein the basic fibroblast growth factor is not contained.
(5) The medium for culturing stem cells described in (1), wherein the growth factor is activin.
(6) The medium for culturing stem cells described in (5), wherein a concentration of the activin is 3 ng/mL or less.
(7) The medium for culturing stem cells described in (5), wherein the activin is not contained.
(8) The medium for culturing stem cells described in any one of (1) to (7), wherein the stem cells are pluripotent stem cells or mesenchymal stem cells.
(9) A method for culturing stem cells, comprising culturing stem cells in the medium for culturing stem cells described in any one of (1) to (8), thereby growing the stem cells having a differentiation ability while maintaining an undifferentiated state of the stem cells.
(10) The method for culturing stem cells described in (9), wherein the stem cells are pluripotent stem cells or mesenchymal stem cells.
(11) A growth promoter for stem cells, comprising at least one or more compounds selected from the group consisting of a ROCK inhibitor, a PKC inhibitor, a histone methyltransferase inhibitor, and a retinoic acid receptor agonist.
(12) The growth promoter for stem cells described in (11), wherein the stem cells are pluripotent stem cells or mesenchymal stem cells.
(13) A method for producing a cell composition containing stem cells, comprising culturing stem cells in the medium for culturing stem cells described in any one of (1) to (8).
(14) The method for producing a cell composition containing stem cells described in (13), wherein the stem cells are pluripotent stem cells or mesenchymal stem cells.
(15) A method for producing a cell composition containing differentiated cells induced from stem cells, comprising culturing stem cells in the medium for culturing stem cells described in any one of (1) to (8) and inducing differentiation of the stem cells.
(16) The method for producing a cell composition containing differentiated cells induced from stem cells described in (15), wherein the stem cells are pluripotent stem cells or mesenchymal stem cells.
(17) A cell composition containing stem cells, produced by the production method described in (13) or (14).
(18) The cell composition described in (17), wherein the stem cells are pluripotent stem cells or mesenchymal stem cells.
(19) A cell composition containing differentiated cells induced from stem cells, produced by the production method described in (15) or (16).
(20) The cell composition described in (19), wherein the stem cells are pluripotent stem cells or mesenchymal stem cells.

### Advantageous Effects of Invention

According to the medium for culturing stem cells of the present invention, by including at least one or more compounds selected from the group consisting of a ROCK inhibitor, a PKC inhibitor, a histone methyltransferase inhibitor, and a retinoic acid receptor agonist, the stem cells can be efficiently cultured while maintaining an undifferentiated state even if the medium does not contain a growth factor or has a low growth factor concentration.

### Brief Description of Drawings

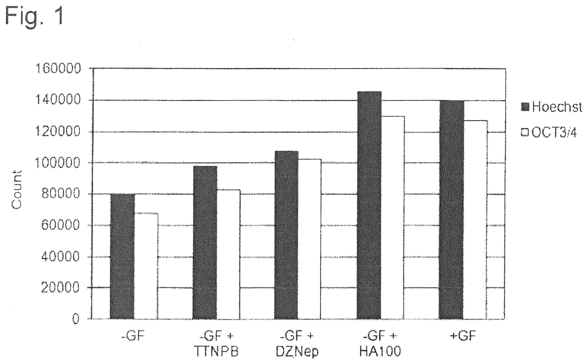

Fig. 1 illustrates the results of counting the number of cell nuclei and the number of cells positive for OCT3/4 protein that is an undifferentiation marker, when culturing human iPS cells using a medium for culturing stem cells of the invention.

### Description of Embodiments

The invention relates to a medium for culturing stem cells (hereinafter also referred to as "the medium of the invention") characterized by containing at least one or more compounds selected from the group consisting of a ROCK inhibitor, a PKC inhibitor, a histone methyltransferase inhibitor, and a retinoic acid receptor agonist and not containing a growth factor or having a low growth factor concentration; a method for culturing stem cells (hereinafter also referred to as "the method of the invention") characterized by including culturing stem cells in the medium, thereby growing the stem cells having a differentiation ability while maintaining an undifferentiated state of the stem cells; a growth promoter for stem cells (hereinafter also referred to as "the growth promoter of the invention"); as well as a cell composition containing stem cells or differentiated cells therefrom; and a method for producing the cell composition.

### 1. Medium of Invention

The medium of the invention is a medium for culturing stem cells containing at least one or more compounds selected from the group consisting of a ROCK inhibitor, a PKC inhibitor, a histone methyltransferase inhibitor, and a retinoic acid receptor agonist and not containing a growth factor or having a low growth factor concentration.

The medium of the invention is a medium capable of growing stem cells while maintaining an undifferentiated state, and is used for culturing stem cells having a differentiation ability.

The "stem cells" as used herein refer to immature cells having a self-replication ability and a differentiation and proliferation ability. There is a hierarchy of stem cells, and it is known that undifferentiated stem cells at a higher class have a high self-replication ability and also have high pluripotency capable of differentiating into various cell lineages, however, as the class goes down, the cells lose the self-replication ability and can differentiate only into a specific cell lineage.

The "pluripotency" as used herein can also be referred to as "pluripotent ability", and refers to an ability to be able to differentiate into any of differentiated cells belonging to ectoderm, mesoderm, and endoderm, and can also include an ability to differentiate into germ cells here. Incidentally, the "differentiated cells" as used herein are many types of cells differentiated from stem cells and constituting tissues or organs in the body.

The origin species of the stem cells to which the medium of the invention can be applied is not particularly limited, and for example, rodents such as rats, mice, hamsters, and guinea pigs, lagomorpha such as rabbits, ungulates such as pigs, cattle, goats, and sheep, carnivora such as dogs and cats, and primates such as humans, monkeys, rhesus monkeys, marmosets, orangutans, and chimpanzees, and the like are exemplified.

The stem cells include pluripotent stem cells, multipotent stem cells, unipotent stem cells, and the like according to the differentiation ability.

The pluripotent stem cells mean cells that have an ability to be able to differentiate into all tissues and cells constituting a living body and are capable of self-replication. The multipotent stem cells mean cells that have an ability to be able to differentiate into a plurality of types, though not all types, of tissues and cells and are capable of self-replication. The unipotent stem cells mean cells that have an ability to be able to differentiate into a specific tissue or cell and are capable of self-replication.

Examples of the pluripotent stem cells can include embryonic stem cells (ES cells), induced pluripotent stem cells (iPS cells), embryonic germ cells (EG cells), embryonal carcinoma cells (EC cells), and adult pluripotent stem cells (APS cells).

Additional examples of the pluripotent stem cells can include stem cells established by culturing an early embryo prepared by somatic nuclear transfer (Nature, 1997, 385, 810, Science, 1998, 280, 1256, Nature Biotechnology, 1999, 17, 456, Nature, 1998, 394, 369, Nature Genetics, 1999, 22, 127, Proc. Natl. Acad. Sci. USA, 1999, 96, 14984, Nature Genetics, 2000, 24, 109).

Examples of human ES cells among the ES cells can include WA01 (HI) and WA09 (H9) (both are available from WiCell Research Institute), and KhES-1, KhES-2, and KhES-3 (all are available from Institute for Frontier Life and Medical Sciences, Kyoto University (Kyoto, Japan)).

Examples of the iPS cells include cells that are obtained by introducing a plurality of genes (reprogramming factors) into somatic cells such as skin cells, and have acquired a pluripotent ability similar to that of ES cells. For example, iPS cells obtained by introducing Oct3/4 gene, Klf4 gene, C-Myc gene, and Sox2 gene, and iPS cells obtained by introducing Oct3/4 gene, Klf4 gene, and Sox2 gene (Nature Biotechnology, 2008, 26, 101-106), and the like are exemplified.

Examples of the gene included in the reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1. These reprogramming factors may be used alone or may be used in combination.

Examples of the combination of the reprogramming factors include combinations described in WO 2007/069666, WO 2008/118820, WO 2009/007852, WO 2009/032194, WO 2009/058413, WO 2009/057831, WO 2009/075119, WO 2009/079007, WO 2009/091659, WO 2009/101084, WO 2009/101407, WO 2009/102983, WO 2009/114949, WO 2009/117439, WO 2009/126250, WO 2009/126251, WO 2009/126655, WO 2009/157593, WO 2010/009015, WO 2010/033906, WO 2010/033920, WO 2010/042800, WO 2010/050626, WO 2010/056831, WO 2010/068955, WO 2010/098419, WO 2010/102267, WO 2010/111409, WO 2010/111422, WO 2010/115050, WO 2010/124290, WO 2010/147395, WO 2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26: 2467-2474, Huangfu D, et al. (2008), Nat Biotechnol. 26: 1269-1275, Shi Y, et al. (2008), Cell Stem Cell, 3, 568-574, Zhao Y, et al. (2008), Cell Stem Cell, 3: 475-479, Marson A, (2008), Cell Stem Cell, 3, 132-135, Feng B, et al. (2009), Nat Cell Biol. 11: 197-203, R. L. Judson et al., (2009), Nat. Biotech., 27: 459-461, Lyssiotis CA, et al. (2009), Proc Natl Acad Sci USA. 106: 8912-8917, Kim JB, et al. (2009), Nature. 461: 649-643, Ichida JK, et al. (2009), Cell Stem Cell. 5: 491-503, Heng JC, et al. (2010), Cell Stem Cell. 6: 167-74, Han J, et al. (2010), Nature. 463: 1096-100, Mali P, et al. (2010), Stem Cells. 28: 713-720, Maekawa M, et al. (2011), Nature. 474: 225-9, etc. The iPS cells are available from a given institute (RIKEN BioResource Center, Kyoto University).

More specific examples of human iPS cells among the iPS cells include 253G1 cell line (RIKEN Cell Bank No. HPS0002), 201B7 cell line (RIKEN Cell Bank No. HPS0063), 409B2 cell line (RIKEN Cell Bank No. HPS0076), 454E2 cell line (RIKEN Cell Bank No. HPS0077), HiPS-RIKEN-1A cell line (RIKEN Cell Bank No. HPS0003), HiPS-RIKEN-2A cell line (RIKEN Cell Bank No. HPS0009), HiPS-RIKEN-12A cell line (RIKEN Cell Bank No. HPS0029), and Nips-B2 cell line (RIKEN Cell Bank No. HPS0223).

Examples of the multipotent stem cells include somatic stem cells such as mesenchymal stem cells, hematopoietic stem cells, neural stem cells, bone marrow stem cells, and germline stem cells. Here, the mesenchymal stem cells mean stem cells capable of differentiating into all or some of mesenchymal cells such as osteoblasts, chondroblasts, or adipoblasts or populations of progenitor cells thereof in a broad sense.

Specific examples of the mesenchymal stem cells include human bone marrow-derived mesenchymal stem cells (hMSC-BM, manufactured by Takara Bio, Inc.), umbilical cord matrix-derived mesenchymal stem cells (hMSC-UC, manufactured by Takara Bio, Inc.), and human adipose tissue-derived mesenchymal stem cells (hMSC-AT, manufactured by Takara Bio, Inc.).

The cells to be cultured in the medium of the invention are preferably cells hierarchically located at a relatively high level, in other words, stem cells in an undifferentiated state and capable of self-replication while sufficiently maintaining pluripotency.

As such stem cells, for example, pluripotent stem cells or multipotent stem cells can be exemplified. The pluripotent stem cells are preferably ES cells or iPS cells, more preferably iPS cells, particularly preferably human iPS cells. The multipotent stem cells are preferably mesenchymal stem cells, more preferably bone marrow mesenchymal stem cells.

The medium of the invention can be prepared, for example, by incorporating at least one or more compounds selected from the group consisting of a ROCK inhibitor, a PKC inhibitor, a histone methyltransferase inhibitor, and a retinoic acid receptor agonist in a minimal essential medium for stem cells. The minimal essential medium for stem cells is not particularly limited as long as it does not contain a growth factor or has a low growth factor concentration.

The minimal essential medium for stem cells preferably contains one or more types of saccharides, one or more types of inorganic salts, one or more types of amino acids, one or more types of vitamins, and one or more types of minor components. Further, it is also possible to contain an antibiotic such as kanamycin as appropriate for using the medium in a drug sensitivity test.

Examples of the saccharides include monosaccharides such as glucose, lactose, mannose, fructose, and galactose, and disaccharides such as sucrose, maltose, and lactose. Among these, glucose is particularly preferred. Among these saccharides, it is also possible to add one saccharide or two or more saccharides in combination.

Examples of the inorganic salts include calcium chloride, calcium nitrate, copper sulfate pentahydrate, iron(III) nitrate nonahydrate, iron(II) sulfate heptahydrate, magnesium chloride hexahydrate, magnesium sulfate, potassium chloride, sodium chloride, disodium hydrogen phosphate, disodium hydrogen phosphate dihydrate, sodium dihydrogen phosphate, sodium dihydrogen phosphate dihydrate, and zinc sulfate heptahydrate.

Examples of the amino acids include alanine, arginine, asparagine, aspartic acid, cystine, cysteine, glutamine, glycine, histidine, glutamic acid, hydroxyproline, isoleucine, leucine, lysine, methionine, phenylalanine, proline, serine, threonine, tryptophan, tyrosine, and valine, and L-amino acids are preferred. In the amino acids, derived materials such as derivatives, salts, and hydrates thereof can be included.

Examples of the derived materials of arginine include L-arginine hydrochloride. Examples of the derived materials of aspartic acid include sodium L-aspartate monohydrate, L-aspartic acid monohydrate, potassium L-aspartate, and magnesium L-aspartate.

Examples of the derived materials of cysteine include L-cysteine dihydrochloride and L-cysteine hydrochloride monohydrate. Examples of the derived materials of lysine include L-lysine hydrochloride. Examples of the derived materials of glutamic acid include monosodium L-glutamate.

Examples of the derived materials of asparagine include L-asparagine monohydrate. Examples of the derived materials of tyrosine include L-tyrosine disodium dihydrate. Examples of the derived materials of histidine include histidine hydrochloride and histidine hydrochloride monohydrate. Examples of the derived materials of lysine include L-lysine hydrochloride.

Examples of the vitamins include ascorbic acid, biotin, choline, folic acid, inositol, niacin, pantothenic acid, pyridoxine, riboflavin, thiamine, vitamin B12, and para-aminobenzoic acid (PABA). Among these, it is preferred to add ascorbic acid. In the vitamins, derived materials such as derivatives, salts, and hydrates thereof can be included.

Examples of the derived materials of ascorbic acid include ascorbic acid 2-phosphate, magnesium ascorbyl phosphate, sodium ascorbyl sulfate, aminopropyl ascorbyl phosphate, and sodium ascorbyl phosphate.

Examples of the derived materials of choline include choline chloride. Examples of the derived materials of niacin include nicotinic acid, nicotinamide, and nicotinic alcohol. Examples of the derived materials of pantothenic acid include calcium pantothenate, sodium pantothenate, and panthenol.

Examples of the derived materials of pyridoxine include pyridoxine hydrochloride, pyridoxal hydrochloride, phosphate pyridoxal, and pyridoxamine. Examples of the derived materials of thiamine include thiamine hydrochloride, thiamine nitrate, bisthiamine nitrate, thiamine dicetyl sulfate ester salt, fursultiamine hydrochloride, octotiamine, and benfotiamine.

The minor components are preferably components that advantageously act on the maintenance of an undifferentiated state of the pluripotent stem cells. Examples of the minor components include components generally used as medium components such as glutathione, hypoxanthine, lipoic acid, linolenic acid, phenol red, putrescine, pyruvic acid, thymidine, and NaHCO₃. In the minor components, derived materials such as derivatives, salts, and hydrates thereof can be included. Examples of the derived materials can include putrescine dihydrochloride.

As the minimal essential medium for stem cells, a minimal essential medium known to those skilled in the art can be used, however, it is preferably a medium, which is easily prepared, and in which the composition of the compounds contained has been revealed so as to prevent the variation between lots.

Examples of such a minimal essential medium for stem cells can include MEM (Minimum Essential Medium), BME (Basal Medium Eagle), DMEM (Dulbecco's Modified Eagle Medium), EMEM (Eagle's minimal essential medium), IMDM (Iscove's Modified Dulbecco's Medium), GMEM (Glas-gow's MEM), F12 (Ham's F12 Medium), RPMI 1640, RD, BMOC-3 (Brinster's BMOC-3 Medium), CMRL-1066, L-15 medium (Leibovitz's L-15 medium), McCoy's 5A, Media 199, MEM αMedia, MCDB 105, MCDB 131, MCDB 153, MCDB 201, and Williams' medium E and ESF.

Additional examples of the minimal essential medium for stem cells can include a medium obtained by mixing any two or more media of the known minimal essential media described above at an appropriate ratio such as DMEM/F12 medium (a medium obtained by mixing DMEM and F12 medium at 1:1), and a medium obtained by adding the ascorbic acid described above, preferably ascorbic acid 2-phosphate to such a medium.

As the minimal essential medium for stem cells, particularly, ESF Nutrient Medium (hereinafter also referred to as "mESF Basal Medium") shown in Miho Furue et al. In Vitro Cell Dev Biol Anim. 41, 19-28 (2005) can be preferably exemplified.

The phrase "not containing a growth factor" as used herein refers to that a growth factor is not substantially contained. Here, the phrase "not substantially contained" means that a growth factor is not intentionally contained in the medium and does not exclude unavoidable contamination with an impurity.

Examples of the growth factor include a basic fibroblast growth factor (bFGF) or the like that is a factor belonging to the fibroblast growth factor (FGF) family, activin (activin A, activin B, or activin AB) or TGF-β₁ or the like that is a factor belonging to the transforming growth factor (TGF)-β family, heregulin-β-1 or the like that is a factor belonging to the neuregulin family, IFG-1 or the like that is a factor belonging to the insulin-like growth factor (IGF) family, and Wnt-3a or the like that is a factor belonging to the Wnt family.

The medium of the invention does not substantially contain a growth factor as described above, but may contain a growth factor at a low concentration. For example, when bFGF is contained in the medium of the invention as a growth factor, the concentration thereof is preferably 2 ng/mL or less, more preferably 1 ng/mL or less.

For example, when activin is contained in the medium of the invention as a growth factor, the concentration thereof is preferably 3 ng/mL or less, more preferably 1 ng/mL or less.

For example, when TGF-β is contained in the medium of the invention as a growth factor, the concentration thereof is preferably 0.06 ng/mL or less, more preferably 0.01 ng/mL or less.

For example, when heregulin-β-1 is contained in the medium of the invention as a growth factor, the concentration thereof is preferably 10 ng/mL or less, more preferably 1 ng/mL or less.

For example, when IFG-1 is contained in the medium of the invention as a growth factor, the concentration thereof is preferably 200 ng/mL or less, more preferably 10 ng/mL or less.

The medium of the invention may contain a growth factor at a low concentration as described above, but preferably does not contain bFGF, TGF-β, activin, heregulin-β-1, IFG-1, or Wnt-3a.

Examples of the ROCK inhibitor include 1-(5-isoquinolinesulfonyl)piperazine hydrochloride (HA100), 1-(5-isoquinolinesulfonyl)homopiperazine dihydrochloride (Fasudil/HA-1077), (S)-(+)-2-methyl-4-glycyl-1-(4-methylisoquinolinyl-5-sulfonyl)homopiperidine dihydrochloride (H-1152), (+)-(R)-trans-4-(1-aminoethyl)-N-(4-pyridyl)cyclohexanecarboxamide dihydrochloride (Y-27632), 1-(5-isoquinolinesulfonyl)-2-methylpiperazine (H-7), 1-(5-isoquinolinesulfonyl)-3-methylpiperazine (iso-H-7), N-2-(methylamino)ethyl-5-isoquinolinesulfonamide dihydrochloride (H-8), N-(2-aminoethyl)-5-isoquinolinesulfonamide dihydrochloride (H-9), N-[2-(p-bromocinnamylamino)ethyl]-5-isoquinolinesulfonamide dihydrochloride (H-89), N-(2-guanidinoethyl)-5-isoquinolinesulfonamide hydrochloride (HA-1004), N-[(LS)-2-hydroxy-1-phenylethyl]-N-[4-(4-pyridinyl)phenyl]-urea (AS 1892802), N-[3-[[2-(4-amino-1,2,5-oxadiazol-3-yl)-1-ethyl-1H-imidazo[4,5-c]pyridin-6-yl]oxy]phenyl]-4-[2-(4-morpholinyl)ethoxy]benzamide (GSK 269962), N-(6-fluoro-1H-indazol-5-yl)-2-methyl-6-oxo-4-(4-(trifluoromethyl)phenyl)-1,4,5,6-tetrahydropyridine-3-carboxamide (GSK 429286), hydroxyfasudil (HA-1100), 2-fluoro-N-[[4-(1H-pyrrolo[2,3-b]pyridin-4-yl)phenyl]methyl]benzenemethanamine (OXA 06), N-[(3-hydroxyphenyl)methyl]-N'-[4-(4-pyridinyl)-2-thiazolyl]urea (RKI 1447), 4-(7-{[(3S)-3-amino-1-pyrrolidinyl]carbonyl}-1-ethyl-1H-imidazole[4,5-c]pyridin-2-yl)-1,2,5-oxadiazol-3-amine (SB 772077B), N-[2-[2-(dimethylamino)ethoxy]-4-(1H-pyrazol-4-yl)phenyl-2,3-dihydro-1,4-benzodioxin-2-carboxamide dihydrochloride (SR 3677), and 6-chloro-N4-[3,5-difluoro-4-[(3-methyl-1H-pyrrolo[2,3-b]pyridin-4-yl)oxy]phenyl]-2,4-pyrimidinediamine (TC-S 7001). Most preferred examples thereof include 1-(5-isoquinolinesulfonyl)piperazine hydrochloride (HA100).

When the ROCK inhibitor is contained in the medium of the invention, the concentration thereof is preferably from 0.001 to 1000 µM, more preferably from 0.01 to 100 µM, most preferably from 0.1 to 10 µM.

Examples of the PKC inhibitor include 1-(5-isoquinolinesulfonyl)piperazine hydrochloride (HA100), 1-(5-isoquinolinesulfonyl)homopiperazine dihydrochloride (Fasudil/HA-1077), (S)-(+)-2-methyl-4-glycyl-1-(4-methylisoquinolinyl-5-sulfonyl)homopiperidine dihydrochloride (H-1152), bisindolylmaleimide II, C-1, calphostin C, 5,6-bis[(4-fluorophenyl)amino]-1H-isoindole-1,3(2H)-dione (CGP 53353), chelerythrine, dihydrosphingosine, S)-(+)-2-methyl-1-[(4-methyl-5-isoquinolinyl)sulfonyl]homopiperazine (H-1152), bisindolylmaleimide I), 12-(2-cyanoethyl)-6,7,12,13-tetrahydro-13-methyl-5-oxo-5H-indolo[2,3-a]pyrrolo[3,4-c]carbazole (Go 6976, 2-[1-(3-dimethylaminopropyl)-5-methoxyindol-3-yl]-3-(1H-indol-3-yl)maleimide (Go 6983), staurosporine aglycone (K-252c), (S)-13-[(dimethylamino)methyl]-10,11,14,15-tetrahydro-4,9:16,21-dimetheno-1H,13H-dibenzo[e,k]pyrrolo[3,4-h][1,4,13]oxadiazacyclohexadecine-1,3(2H)-dione (LY 333531), melittin, palmitoyl carnitine, 2-{8-[(dimethylamino)methyl]-6,7,8,9-tetrahydropyrido[1,2-a]indol-3-yl}-3-(1-methyl-1H-indol-3-yl)maleimide (Ro-32-0432), rottlerin, E-erythrosphingosine, and 3-[5-[4-(2-hydroxy-2-methyl-1-oxopropyl)-1-piperazinyl]-2-(trifluoromethyl)phenyl]-4-(1H-indol-3-yl)-1H-pyrrole-2,5-dione (TCS 21311), and most preferred examples thereof include 1-(5-isoquinolinesulfonyl)piperazine hydrochloride (HA100).

When the PKC inhibitor is contained in the medium of the invention, the concentration thereof is preferably from 0.001 to 1000 µM, more preferably from 0.01 to 100 µM, most preferably from 0.1 to 10 µM.

Among the compounds shown as examples of the ROCK inhibitor and the PKC inhibitor, HA100, Fasudil/HA-1077, and H-1152 can function as both the ROCK inhibitor and the PKC inhibitor.

Examples of the histone methyltransferase inhibitor include 3-deazaneplanocin (DzNep), N-[(6-methyl-2-oxo-4-propyl-1H-pyridin-3-yl)methyl]-1-propan-2-yl-6-[6-(4-propan-2-ylpiperazin-1-yl)pyridin-3-yl]indazole-4-carboxamide (UNC 1999), and Sinefungin, and most preferred examples thereof include 3-deazaneplanocin (DzNep).

When the histone methyltransferase inhibitor is contained in the medium of the invention, the concentration thereof is preferably from 0.0001 to 10 µM, more preferably from 0.001 to 1 µM, most preferably from 0.001 to 0.1 µM.

Examples of the retinoic acid receptor agonist include 4-[(E)-2-(5,6,7,8-tetrahydro-5,5,8,8-tetramethyl-2-naphthalenyl)-1-propenyl]benzoic acid (TTNPB), 4-[4-(2-butoxyethoxy-)-5-methyl-2-thiazolyl]-2-fluorobenzoic acid (AC 261066), adapalene, 4-[(5,6,7,8-tetrahydro-5,5,8,8-tetramethyl-2-naphthalenyl)carboxamido]benzoic acid (AM 580), Tamibarotene (AM 80), 4-[[(2,3-dihydro-1,1,3,3-tetramethyl-2-oxo-1H-inden-5-yl)carbonyl]amino]benzoic acid (BMS 753), 1-(4-hexaphenyl)-2-propan-1-one, 3-fluoro 4-[[2-hydroxy-2-(5,5,8,8-tetramethyl-5,6,7,8-tetrahydro-2-naphthalenyl)acetyl]amino]-benzoic acid (BMS 961), 4-(6-hydroxy-7-tricyclo[3.3.1.13,7]dec-1-yl-2-naphthalenyl)benzoic acid (CD 1530), 5-(5,6,7,8-tetrahydro-5,5,8,8-tetramethyl-2-anthracenyl)-3-thiophenecarboxylic acid (CD 2314), 6-[3-(1-adamantyl)-4-hydroxyphenyl]-2-naphthalenecarboxylic acid (CD 437), (E)-4-[3-(3,5-di-tert-butylphenyl)-3-oxo-1-propenyl]benzoic acid (Ch55), isotretinoin, retinoic acid, and tazarotene, and most preferred examples thereof include 4-[(E)-2-(5,6,7,8-tetrahydro-5,5,8,8-tetramethyl-2-naphthalenyl)-1-propenyl]benzoic acid (TTNPB).

When the retinoic acid receptor agonist is contained in the medium of the invention, the concentration thereof is preferably from 0.001 pM to 10 nM, more preferably from 0.01 pM to 1 nM, most preferably from 0.01 pM to 0.1 nM.

The medium of the invention contains at least one or more compounds selected from the group consisting of a ROCK inhibitor, a PKC inhibitor, a histone methyltransferase inhibitor, and a retinoic acid receptor agonist.

That is, in the medium of the invention, one or more compounds of any of the ROCK inhibitor, the PKC inhibitor, the histone methyltransferase inhibitor, and the retinoic acid receptor are contained, and for example, one type or two or more types of compounds included in the ROCK inhibitor may be contained, or compounds selected from two or more types of the ROCK inhibitor, the PKC inhibitor, the histone methyltransferase inhibitor, and the retinoic acid receptor may be contained. When compounds selected from two or more types of the ROCK inhibitor, the PKC inhibitor, the histone methyltransferase inhibitor, and the retinoic acid receptor are contained, the concentrations thereof are preferably concentrations defined above for the respective compounds.

Further, to the medium of the invention, insulin, transferrin, albumin, a lipid group, 2-mercaptoethanol, ethanolamine, sodium selenite, a nucleic acid, a nonessential amino acid, or the like may be added as needed. Further, if necessary, an arbitrary component such as a pH adjusting agent, a buffer component, a humectant, an antiseptic, or a viscosity modifier can also be used.

Examples of a method for confirming whether or not cells cultured in the medium of the invention are cells that can grow while maintaining an undifferentiated state include a method for determination by detection of the expression of an undifferentiation marker (protein) and/or non-detection of the expression of a differentiation marker (protein) using antibodies against various types of markers (proteins), a method for determination by detection of the expression of various types of undifferentiation markers (genes) and/or non-detection of differentiation markers (genes), a method for observing the morphological features of cells, a method for determining whether or not an ability to differentiate into a specific cell by stimulation with a specific differentiation inducing factor can be exhibited, a method for transplanting cells into a mouse to form a teratoma and carrying out pathological observation of histomorphology, and a method for forming embryoid bodies (EBs) and inducing spontaneous differentiation.

As the method for determination using a marker, for example, when an undifferentiation marker such as NANOG, OCT3/4, SSEA-3, SSEA-4, TRA-2-54, TRA-1-60, TRA-1-80, CD90, or alkaline phosphatase is expressed, it can be determined that stem cells cultured in the medium of the invention maintain an undifferentiated state.

When the expression of an undifferentiation marker and/or a differentiation marker is confirmed at a protein level, the confirmation can be carried out, for example, by flow cytometry, immunostaining, ELISA, or the like using an antibody specific to each marker (protein).

When the expression of an undifferentiation marker and/or a differentiation marker is confirmed at a gene level, the confirmation can be carried out, for example, by RT-PCR using a primer pair specific to each marker (gene) or Northern blotting, microarray analysis, or the like using a probe specific to each marker (gene).

Examples of a method for confirming the presence or absence of the expression of an undifferentiation marker and/or a differentiation marker by flow cytometry include the following method.

Cells cultured in the medium of the invention are suspended in 1 mL of 10% goat serum for 30 minutes, followed by centrifugation, and thereafter, the cells are incubated for 30 minutes together with a mouse antibody against a target marker (protein). Thereafter, the cultured cells are washed three times with PBS containing 1% goat serum, and reacted with a fluorescently labeled goat anti-mouse IgG antibody for 30 minutes, and then washed three times with PBS containing 1% goat serum. The resuspended cells are determined using an appropriate apparatus for flow cytometry.

Examples of a method for confirming the presence or absence of the expression of an undifferentiation marker and/or a differentiation marker by immunostaining include the following method.

Cells cultured in the medium of the invention are fixed with 4% paraformaldehyde (PFA) in PBS, and then washed a plurality of times with PBS. Thereafter, blocking is carried out with 10% goat serum or bovine-derived albumin, and then, immunostaining is carried out using a mouse antibody against a target marker (protein). Then, the cells are reacted with fluorescent label (AlexaFluor)-conjugated goat anti-mouse IgG, and determined by fluorescence microscopy and an image analyzer. Incidentally, when a marker (protein) is present in a cytoplasm, the permeability of the cultured cells is increased with Triton X-100 or the like, and immunostaining is carried out using an antibody.

Examples of a method for confirming the presence or absence of the expression of alkaline phosphatase include so-called alkaline phosphatase staining.

Cells cultured in the medium of the invention are fixed for 5 minutes with 4.5 mM citric acid, 2.25 mM sodium citrate, 3 mM sodium chloride, 65% methanol, and 4% paraformaldehyde, followed by washing, and then, alkaline phosphatase is visualized by using an appropriate kit such as FastRed Substrate Kit (manufactured by Sigma Co.), whereby confirmation can be carried out.

### 2. Method of Invention

The method of the invention is characterized by including culturing stem cells in the medium of the invention, thereby efficiently growing the stem cells having a differentiation ability while maintaining an undifferentiated state of the stem cells.

Whether or not stem cells can be grown while maintaining an undifferentiated state can be confirmed by the method for determination by detection of the expression of an undifferentiation marker (protein) and/or non-detection of the expression of a differentiation marker (protein) using antibodies against various types of markers (proteins), or the like described above.

The definition of "stem cells" is as described in the above 1.

By using the medium of the invention, stem cells can be cultured by batch culture (Bach Culture), fed-batch culture (Fed-Bach Culture), continuous culture, perfusion culture, or the like.

A culture device to be used for culture is not particularly limited, and examples thereof include a flask, a dish, a plate, a microwell plate, a microslide, a chamber slide, a tube, a tray, and a culture bath such as a culture bag or tank. A substrate of such a culture device is also not particularly limited, and examples thereof include glass, various types of plastics such as polypropylene and polystyrene, metals such as stainless steel, and a combination thereof.

When culturing stem cells in the medium of the invention, the method of the invention can be used in any culture of adhesion culture and floating culture.

When carrying out adhesion culture, the culture can be carried out by coating the surface of the substrate with a scaffold such as collagen, gelatin, poly-L-lysine, poly-D-lysine, laminin, a partial structure of laminin, fibronectin, a mixture thereof (for example, Matrigel), or a lysed cell membrane preparation (Lancet, 2005, 365, pp. 1636-1641) for inducing the adhesion of stem cells to the surface of the substrate of the culture device.

Further, the culture may be carried out in the presence of feeder cells. As the feeder cells, stromal cells such as fetal fibroblasts can be used (see, for example, Manipulating the Mouse Embryo A Laboratory Manual, Second Edition, Cold Spring Harbor Laboratory Press, 1994, Gene Targeting, A Practical Approach, IRL Press at Oxford University Press, 1993, Proc. Natl. Acad. Sci. USA, 1981, 78, 12, pp. 7634-7638, Nature, 1981, 292, 5819, pp. 154-156, J. Virol., 1969, 4, 5, pp. 549-553, Science, 1996, 272, 5262, pp. 722-724, J. Cell. Physiol., 1982, 112, 1, pp. 89-95, WO 01/088100, and WO 2005/080554).

When carrying out floating culture, for example, if a high molecular weight polymer such as methyl cellulose (Stem Cell Reports, 2014, 2, 5, 734-745), MFG-E8 (Milk fat globule-EGF factor 8), or a fragment of the protein, or the like is used, the above-mentioned scaffold is no longer needed.

The culture density of stem cells is not particularly limited as long as the density can achieve an effect of promoting the survival and growth of the cells. The culture density is, for example, generally from 1.0×10¹ to 1.0×10⁷ cells/mL, preferably from 1.0×10² to 1.0×10⁷ cells/mL, more preferably from 1.0×10³ to 1.0×10⁷ cells/mL, further more preferably from 3.0×10⁴ to 1.0×10⁷ cells/mL. The culture conditions such as a temperature, a dissolved CO₂ concentration, a dissolved oxygen concentration, and a pH can be appropriately set based on a technique conventionally used for the culture of cells derived from an animal tissue.

For example, the culture is preferably carried out at generally 33 to 40°C, preferably 34 to 39°C, more preferably 36 to 37°C, in the presence of carbon dioxide at generally 1 to 20%, preferably 3 to 15%, more preferably 5 to 10% at a high humidity of generally 75% or more, preferably 85% or more, more preferably 95% or more, particularly preferably 100%.

According to the method of the invention, it is possible to promote the survival and growth of stem cells in a medium containing at least one or more compounds selected from the group consisting of a ROCK inhibitor, a PKC inhibitor, a histone methyltransferase inhibitor, and a retinoic acid receptor agonist and not containing a growth factor or having a low growth factor concentration. Therefore, the method of the invention can be utilized particularly for efficiently culturing stem cells known to be vulnerable to stress due to dispersion and floating culture or the like after dispersion.

### 3. Growth Promoter of Invention

The growth promoter of the invention contains at least one or more compounds selected from the group consisting of a ROCK inhibitor, a PKC inhibitor, a histone methyltransferase inhibitor, and a retinoic acid receptor agonist.

By culturing stem cells using a medium containing the growth promoter of the invention, the growth efficiency of the stem cells is dramatically enhanced even if the medium does not contain a growth factor or has a low growth factor concentration.

The growth promoter of the invention may further contain, for example, a physiological isotonic solution [an isotonic solution containing physiological saline, any of the above-mentioned minimal essential media, glucose, or another adjuvant (for example, D-sorbitol, D-mannitol, sodium chloride, or the like), etc.], an excipient, an antiseptic, a stabilizing agent (for example, human serum albumin, polyethylene glycol, etc.), a binder, a solubilizing aid, a nonionic surfactant, a buffer (for example, a phosphate buffer solution or a sodium acetate buffer solution), a preservative, an antioxidant, any of the above-mentioned additives, or the like other than the ROCK inhibitor, the PKC inhibitor, the histone methyltransferase inhibitor, and the retinoic acid receptor agonist.

The content of at least one or more compounds selected from the group consisting of the ROCK inhibitor, the PKC inhibitor, the histone methyltransferase inhibitor, and the retinoic acid receptor agonist contained in the growth promoter of the invention is preferably configured such that when the growth promoter of the invention is added to a medium, the concentration of each compound in the medium is a concentration sufficient for promoting the growth of stem cells, that is, each compound is contained at a concentration described in the above 1.

The growth promoter of the invention is preferably used in a method of adding it to a medium in a state of an isotonic aqueous solution, a powder, or another form, or the like.

### 4. Method for Producing Cell Composition Containing Stem Cells or Differentiated Cells Therefrom of Invention

The method for producing a cell composition containing stem cells or differentiated cells therefrom of the invention is the method for producing a cell composition containing stem cells of the invention, or the method for producing a cell composition containing differentiated cells induced from stem cells of the invention.

The method for producing a cell composition containing stem cells of the invention includes a step of culturing stem cells in the medium of the invention, and includes a step of subculturing the stem cells as needed.

Further, the method for producing a cell composition containing differentiated cells induced from stem cells of the invention includes a step of culturing stem cells in the medium of the invention and a step of inducing differentiation of the stem cells, and includes a step of subculturing the cells as needed.

The subculture or the induction of differentiation of the stem cells may be carried out by a conventionally known method. Examples of a differentiation inducing agent include a BMP inhibitor, a Wnt inhibitor, a Nodal inhibitor, and retinoic acid. The induction of differentiation can be carried out, for example by culturing stem cells in a differentiation induction medium (90% αMEM medium, 10% fetal bovine serum (FBS), 2 mM L-glutamine, 0.1 µM dexamethasone) in a process for inducing differentiation of chondrocytes.

In the method for producing a cell composition containing stem cells or differentiated cells therefrom of the invention, the survival and growth of stem cells can be promoted by the step of culturing stem cells in the presence of at least one or more compounds selected from the group consisting of a ROCK inhibitor, a PKC inhibitor, a histone methyltransferase inhibitor, and a retinoic acid receptor agonist, and therefore, a cell composition containing stem cells or differentiated cells therefrom can be efficiently produced.

### 5. Cell Composition Containing Stem Cells or Differentiated Cells Therefrom of Invention

The cell composition containing stem cells or differentiated cells therefrom of the invention is the cell composition containing stem cells of the invention, or the cell composition containing differentiated cells induced from stem cells of the invention.

The cell composition containing stem cells of the invention is a cell composition containing stem cells produced by the method described in the above 4.

The cell composition containing differentiated cells induced from stem cells of the invention is a cell composition containing differentiated cells induced from stem cells produced by the method described in the above 4.

The cell composition containing stem cells or differentiated cells therefrom of the invention can be utilized for further culturing the stem cells or for a cell source for regenerative medicine.

The cell composition containing stem cells or differentiated cells therefrom of the invention can be used again for subculture of the stem cells or the differentiated cells therefrom by, for example, being dispensed, and then cryopreserved, transported, and thereafter thawed.

When being cryopreserved, the cell composition containing stem cells or differentiated cells therefrom of the invention may further contain serum or a substitute thereof or an organic solvent such as DMSO.

### Examples

Next, the invention is described in more detail with reference to Examples, but the invention is by no means limited thereto.

### [Example 1]

In order to grow stem cells in a medium, it is generally necessary to use a medium containing a growth factor, however, it was confirmed whether or not stem cells can grow using a medium in which various types of compounds were added to a medium not containing a growth factor.

A medium obtained by adding and dissolving separate components shown in the following Table 1 sterilized through a 0.22 µm filter, 10 µg/mL insulin (manufactured by Sigma-Aldrich Co. LLC.), 5 µg/mL apotransferrin (manufactured by Sigma-Aldrich Co. LLC.), and oleic acid-conjugated recombinant human serum albumin (manufactured by Sigma-Aldrich Co. LLC.) in a known medium (mESF Basal Medium, manufactured by Wako Pure Chemical Industries, Ltd.) was used as a medium in a negative control group.

Here, the oleic acid-conjugated recombinant human serum albumin was prepared by adding 235 µL of 200 mg/mL oleic acid (manufactured by Sigma-Aldrich Co. LLC.) to 50 mL of 100 mg/mL recombinant human serum albumin (rHSA, manufactured by Sigma-Aldrich Co. LLC.), followed by mixing.

**[Table 1]**

| Component | Final concentration |
|---|---|
| 2-Mercaptoethanol | 10 µM |
| Sodium selenite | 20 µM |
| Ethanolamine | 10 µM |
| Ascorbic acid 2-phosphate (013-19641, manufactured by Wako Pure Chemical Industries, Ltd.) | 0.1mg/mL |

A medium obtained by adding 5 µM HA100 that is a ROCK inhibitor and a PKC inhibitor, 0.05 µM DZNep that is a histone methyltransferase inhibitor, or 0.01 nM TTNPB that is a retinoic acid receptor agonist to the medium of the negative control group was used as a medium in an evaluation target group.

Further, a medium obtained by adding 2 ng/mL activin A (manufactured by Wako Pure Chemical Industries, Ltd.) and 5 ng/mL bFGF (manufactured by Wako Pure Chemical Industries, Ltd.) to the medium of the negative control group was used as a medium of a positive control group.

The prepared medium in the negative control group, media in the evaluation target group (the medium obtained by adding HA100 to the medium in the negative control group, the medium obtained by adding DZNep to the medium in the negative control group, and the medium obtained by adding TTNPB to the medium in the negative control group,), and medium in the positive control group were added to a 24-well plate for cell culture (manufactured by Sumitomo Bakelite Company Limited) at 1 mL/well, and a human iPS cell 253G1 cell line (established by Center for iPS Cell Research and Application, Kyoto University and obtained from iPS Academia Japan, Inc.) was cultured in the presence of 10% CO₂ at 37°C for 3 days. With respect to the cultured cells, the nuclei were stained with Hoechst 33342 (manufactured by Dojindo Laboratories). Further, OCT3/4 protein in the cells was stained with OCT-3/4 antibody C-10 (manufactured by Santa Cruz Biotechnology) and Chickin anti-mouse IgG (H+L) Secondary Antibod Alexa Fluor 488 conjugate (manufactured by Invitorogen Co.). Thereafter, the number of stained nuclei and the number of Oct3/4 positive cells were counted using In cell analyzer (manufactured by GE Healthcare, Inc.). The average value of the results of four independent experiments is shown in FIG. 1.

When the medium to which no growth factor was added was used, the human iPS cells almost did not grow as compared with the cells at the time of inoculation, however, as shown in FIG 1, in the case of using the medium to which HA100 was added (-GF+HA100), the case of using the medium to which DZNep was added (-GF+DZNep), and the case of using the medium to which TTNPB was added (-GF+TTNPB), the number of nuclei counted was increased to 1.83 times, 1.35 times, and 1.24 times, respectively, as compared with that in the case of using the medium to which no growth factor was added (-GF), and it was shown that the cells grow. In particular, in the medium to which HA100 was added, growth comparable to that in the case of using the medium to which a growth factor was added (+GF) was exhibited.

Further, the expression ratio of OCT3/4 that is the undifferentiation marker of iPS cells cultured in these media was 89.3% in the case of using the medium to which HA100 was added (-GF+HA100), 95.6% in the case of using the medium to which DZNep was added (-GF+DZNep), and 84.2% in the case of using the medium to which TTNPB was added (-GF+TTNPB), and was equal to or more than that in the case of using the medium to which a growth factor was added (+GF, 88.1%). Here, the expression ratio indicates the proportion of the cells positive for Oct3/4 among the cells whose nuclei were stained with Hoechst 33342.

From the results, it was found that according to the medium for culturing stem cells of the invention, stem cells can be efficiently cultured while maintaining an undifferentiated state.

While the present invention has been described in detail with reference to specific embodiments, it is apparent to those skilled in the art that various changes and modifications can be made without departing from the spirit and scope of the present invention. The present application is based on Japanese Patent Application (Japanese Patent Application No. 2016-175941) filed on September 8, 2016 and the entire contents of which are incorporated herein by reference. Further, all references cited herein are incorporated herein by reference in their entirety.

## Claims

1. A medium for culturing stem cells, comprising at least one or more compounds selected from the group consisting of a ROCK (Rho-associated coiled-coil forming kinase) inhibitor, a PKC (protein kinase C) inhibitor, a histone methyltransferase inhibitor, and a retinoic acid receptor agonist and not containing a growth factor or having a low growth factor concentration.

2. The medium for culturing stem cells according to claim 1, wherein the growth factor is a basic fibroblast growth factor.

3. The medium for culturing stem cells according to claim 2, wherein a concentration of the basic fibroblast growth factor is 2 ng/mL or less.

4. The medium for culturing stem cells according to claim 2, wherein the basic fibroblast growth factor is not contained.

5. The medium for culturing stem cells according to claim 1, wherein the growth factor is activin.

6. The medium for culturing stem cells according to claim 5, wherein a concentration of the activin is 3 ng/mL or less.

7. The medium for culturing stem cells according to claim 5, wherein the activin is not contained.

8. The medium for culturing stem cells according to any one of claims 1 to 7, wherein the stem cells are pluripotent stem cells or mesenchymal stem cells.

9. A method for culturing stem cells, comprising culturing stem cells in the medium for culturing stem cells according to any one of claims 1 to 8, thereby growing the stem cells having a differentiation ability while maintaining an undifferentiated state of the stem cells.

10. The method for culturing stem cells according to claim 9, wherein the stem cells are pluripotent stem cells or mesenchymal stem cells.

11. A growth promoter for stem cells, comprising at least one or more compounds selected from the group consisting of a ROCK inhibitor, a PKC inhibitor, a histone methyltransferase inhibitor, and a retinoic acid receptor agonist.

12. The growth promoter for stem cells according to claim 11, wherein the stem cells are pluripotent stem cells or mesenchymal stem cells.

13. A method for producing a cell composition containing stem cells, comprising culturing stem cells in the medium for culturing stem cells according to any one of claims 1 to 8.

14. The method for producing a cell composition containing stem cells according to claim 13, wherein the stem cells are pluripotent stem cells or mesenchymal stem cells.

15. A method for producing a cell composition containing differentiated cells induced from stem cells, comprising culturing stem cells in the medium for culturing stem cells according to any one of claims 1 to 8 and inducing differentiation of the stem cells.

16. The method for producing a cell composition containing differentiated cells induced from stem cells according to claim 15, wherein the stem cells are pluripotent stem cells or mesenchymal stem cells.

17. A cell composition containing stem cells, produced by the production method according to claim 13 or 14.

18. The cell composition according to claim 17, wherein the stem cells are pluripotent stem cells or mesenchymal stem cells.

19. A cell composition containing differentiated cells induced from stem cells, produced by the production method according to claim 15 or 16.

20. The cell composition according to claim 19, wherein the stem cells are pluripotent stem cells or mesenchymal stem cells.
